# FASCICULE DE BREVET EUROPEEN

(11) **EP 2 106 754 B1**
(45) Date de publication et mention de la délivrance du brevet: **01.09.2010**
(21) Numéro de dépôt: 09157095.2
(22) Date de dépôt: 01.04.2009
(51) Int. Cl.: A61B 17/064, A61B 19/00

(54) **Agrafe de compression**
Kompressionsklammer
Compression staple

(30) Priorité: 02.04.2008 FR 0852184
(43) Date de publication de la demande: 07.10.2009
(73) Titulaire: Tornier, 38330 Saint Ismier (FR)
(72) Inventeur: Lizee, Emmanuel, 38330, SAINT ISMIER (FR)
(74) Mandataire: Grand, Guillaume

(56) Documents cités:
- EP-A- 1 284 123
- FR-A- 2 874 166
- FR-A- 2 901 119
- US-A1- 2005 256 537

## Description

La présente invention a trait à une agrafe de compression.

Une agrafe de compression est utilisée pour maintenir en position relative deux parties d'un os en vue de leur consolidation. Une telle agrafe comprend classiquement deux branches reliées par un pont transversal, les branches étant destinées à être insérées dans les parties osseuses de part et d'autre du foyer de fracture ou d'ostéotomie d'un os à réparer. Une telle agrafe comprend en outre des moyens de rapprochement des deux branches l'une par rapport à l'autre, ce qui permet d'appliquer les deux parties osseuses l'une contre l'autre avec une certaine pression.

En particulier, EP-A-0 955 011 décrit une agrafe de compression dans laquelle le rapprochement des branches après implantation de l'agrafe est obtenu par une déformation du pont transversal de l'agrafe. Dans ce dispositif, le pont de l'agrafe forme un oeil dont la déformation en écartement, perpendiculairement à l'axe du pont, permet un raccourcissement du pont et un rapprochement des branches. Toutefois, une fois déformé, le pont présente un encombrement important, perpendiculairement au plan défini par les branches de l'agrafe. Cet encombrement du pont perpendiculairement au plan des branches en configuration implantée de l'agrafe dans un os est encore accentué par la forme cylindrique de l'os, et est susceptible de créer une gêne sous la peau et d'entraîner un endommagement des tissus au voisinage de l'os. De plus, la déformation du pont est actionnée en exerçant une pression axiale sur les extrémités des branches qui émergent de la matière osseuse. A cet effet, ces extrémités des branches sont conçues pour dépasser nettement au-dessus de la surface de l'os. Un tel agencement de l'agrafe en saillie par rapport à la surface de l'os renforce le risque de gêne sous la peau et d'endommagement des tissus.

US-A-2005/256537 (le préambule de la revendication 1 est basé sur ce document) propose, quant à lui, une agrafe de compression comportant deux parties de branche à ancrer dans un os, qui sont reliées par un pont transversal, depuis la partie centrale duquel s'étend une unique partie de branche, avec interposition de pontets sécables. Pour entraîner les deux parties de branche à ancrer dans l'os lors de leur mise en place, il est nécessaire d'utiliser des bras d'actionnement spécifiques, qui agissent directement sur ces parties de branche. La pose de cette agrafe s'avère ainsi fastidieuse.

Le but de la présente invention est de proposer une agrafe de compression qui, tout en présentant un profil bas et compact en configuration implantée de l'agrafe dans un os, est facile à poser.

A cet effet, l'invention a pour objet une agrafe de compression telle que définie à la revendication 1.

D'autres caractéristiques avantageuses d'une agrafe de compression conforme à l'invention, prises isolément ou selon toutes les combinaisons techniquement possibles, sont spécifiées aux revendications dépendantes 2 à 14.

Est également décrite ici une méthode de pose d'une agrafe de compression telle que décrite ci-dessus pour la consolidation de deux parties osseuses, comprenant des étapes dans lesquelles :
- on perce un alésage dans chacune des deux parties osseuses ;
- on insère la première partie de l'une des branches de l'agrafe dans l'alésage de l'une des parties osseuses et la première partie de l'autre branche dans l'alésage de l'autre partie osseuse, jusqu'à la venue en contact d'un bord du pont, qui est dirigé du côté des premières parties des branches, contre la surface de l'os ;
- on sollicite les premières parties des deux branches de l'agrafe en convergence l'une vers l'autre en exerçant sur la deuxième partie d'au moins l'une des branches un effort de divergence par rapport à la deuxième partie de l'autre branche ;
- on sépare la deuxième partie de chaque branche de l'agrafe par rapport à la première partie de la branche en obtenant la rupture des moyens sécables.

De manière avantageuse, on perce les alésages dans les deux parties osseuses agencées en appui l'une contre l'autre avec une distance entre les alésages supérieure ou égale à l'entraxe entre les premières parties des branches en configuration parallèle des premières parties l'une par rapport à l'autre.

Une telle méthode de pose peut alors comprendre une étape supplémentaire dans laquelle, préalablement à l'insertion des premières parties des branches de l'agrafe dans les alésages des parties osseuses, on fait diverger les premières parties des deux branches en exerçant sur la deuxième partie d'au moins l'une des branches un effort de convergence vers la deuxième partie de l'autre branche.

Les caractéristiques et avantages de l'invention apparaîtront dans la description qui va suivre d'un mode de réalisation d'une agrafe de compression selon l'invention, donnée uniquement à titre d'exemple et faite en se référant aux dessins annexés dans lesquels :
- la figure 1 est une vue en perspective d'une agrafe de compression conforme à l'invention ;
- la figure 2 est une vue de face de l'agrafe de compression de la figure 1 ;
- la figure 3 est une vue partielle à plus grande échelle selon la flèche III de la figure 1 ;
- la figure 4 est une vue de face de l'agrafe de compression des figures 1 à 3, lors d'une première étape d'implantation de l'agrafe dans un os ;
- la figure 5 est une vue analogue à la figure 4, lors d'une deuxième étape d'implantation de l'agrafe dans un os ; et
- la figure 6 est une vue analogue à la figure 4, en configuration implantée de l'agrafe dans un os.

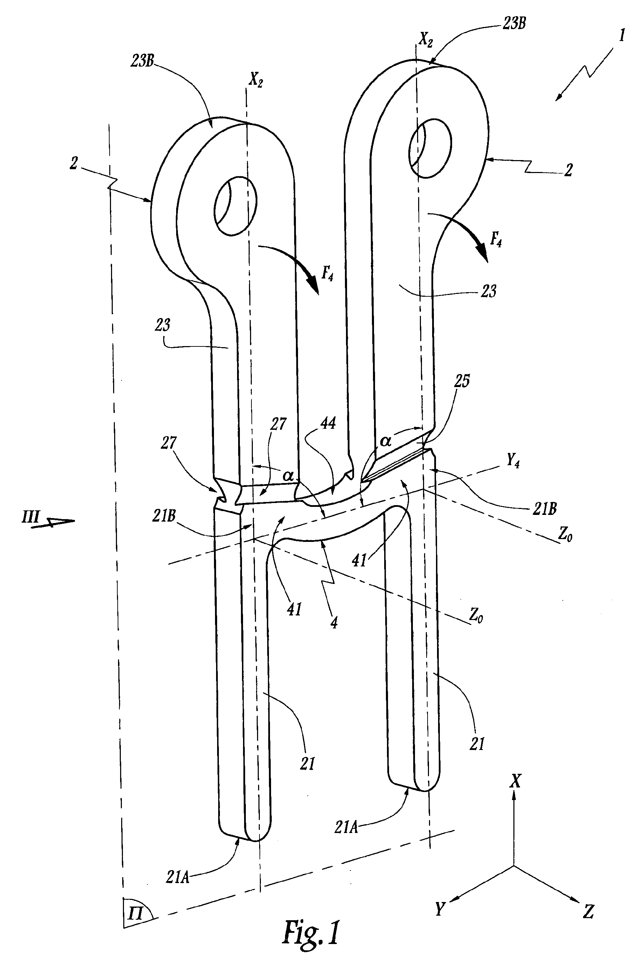

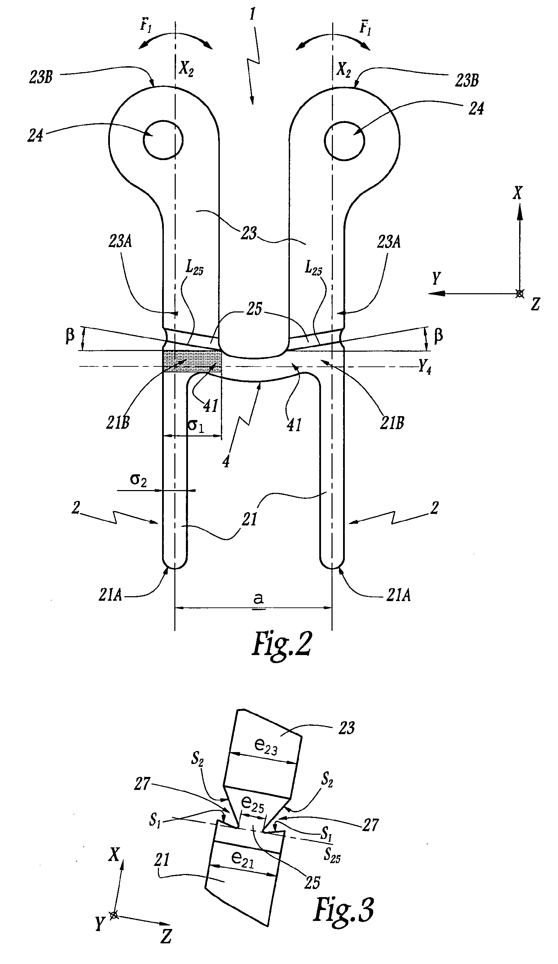

L'agrafe de compression 1 représentée sur la figure 1 comprend deux branches 2 de forme allongée et un pont transversal 4 reliant les deux branches. L'agrafe 1 est formée par une plaque en acier inoxydable découpée globalement selon une forme de H, de manière à définir les deux branches 2 et le pont 4 raccordé à une portion médiane de chaque branche 2. En variante, l'agrafe 1 peut être constituée en tout autre matériau approprié, par exemple en titane. L'agrafe 1 est destinée à la consolidation de deux parties d'un os, suite à une fracture ou à une ostéotomie. On note X une direction longitudinale de l'agrafe 1, Y une direction transversale de l'agrafe 1 et Z une direction de profondeur de l'agrafe 1. Préalablement à son implantation dans un os, l'agrafe 1 est dans une configuration initiale montrée sur les figures 1 et 2, dans laquelle les axes longitudinaux X₂ des deux branches 2 sont sensiblement parallèles entre eux, et à la direction X, et sont perpendiculaires à l'axe longitudinal Y₄ du pont 4, lequel est parallèle à la direction Y.

Grâce aux propriétés mécaniques du matériau constitutif de l'agrafe 1, chaque branche 2 de l'agrafe 1 est apte à être déplacée en pivotement par rapport à l'extrémité 41 adjacente du pont 4, de part et d'autre de sa position perpendiculaire au pont 4 visible sur les figures 1 et 2, comme montré par les doubles flèches F₁ de la figure 2, en restant dans un plan moyen de l'agrafe 1 défini par l'axe longitudinal X₂ de chaque branche 2 et l'axe longitudinal Y₄ du pont 4. Ainsi, chaque branche 2 est apte à être pivotée par rapport au pont 4 autour d'un axe Zo perpendiculaire au plan moyen π, en restant dans le plan moyen π, entre une position dans laquelle l'angle α délimité entre l'axe longitudinal Y₄ du pont 4 et l'axe longitudinal X₂ de la branche 2 est de l'ordre de 80° et une position dans laquelle l'angle α est de l'ordre de 110°.

Chaque branche 2 de l'agrafe 1 comporte deux parties allongées, agencées dans le prolongement l'une de l'autre, à savoir une partie 21 destinée à être ancrée dans un os et une partie d'entraînement 23 destinée à être actionnée pour l'entraînement en pivotement de la branche 2 par rapport au pont 4, parallèlement au plan moyen π. Au sens de l'invention, deux parties dans le prolongement l'une de l'autre peuvent être agencées dans l'alignement l'une de l'autre ou en biais l'une par rapport à l'autre. Les parties 21 et 23 sont reliées l'une à l'autre par un pontet sécable 25, qui raccorde une extrémité 21B de la partie 21 avec une extrémité 23A de la partie 23. L'épaisseur e₂₅ du pontet sécable 25 selon la direction de profondeur Z de l'agrafe 1 est inférieure à l'épaisseur e₂₁ de la partie 21 et à l'épaisseur e₂₃ de la partie 23 selon la direction Z. En particulier, dans ce mode de réalisation, les épaisseurs e₂₁ et e₂₃ des parties 21 et 23 sont identiques, alors que l'épaisseur e₂₅ du pontet sécable 25 est inférieure à la moitié de l'épaisseur e₂₁ ou e₂₃ des parties 21 et 23. Le pontet 25 est ainsi sécable sous l'action d'un effort F₄ dirigé selon la direction de profondeur Z de l'agrafe 1, c'est-à-dire transversalement par rapport au plan moyen π, et exercé au voisinage de l'extrémité libre de l'une des parties 21 ou 23 de la branche alors que l'autre partie est immobilisée. Tel que représenté sur la figure 1, l'effort F₄ est exerce au voisinage de l'extrémité libre 23B de chaque partie 23, alors que chacune des parties 21 est immobilisée.

Chacune des deux extrémités 41 du pont transversal 4 de l'agrafe 1 est liée à l'extrémité 21B de la partie 21 d'une branche 2, de sorte que chaque extrémité 41 du pont 4 est adjacent au pontet sécable 25 d'une branche 2. Comme visible sur les figures 1 et 2, préalablement à l'implantation de l'agrafe 1 dans un os, le pont 4 de l'agrafe 1 est bombé du côté des parties 21 des branches 2 destinées à être ancrées dans un os. En d'autres termes, un bord 44 du pont 4 dirigé vers les parties 23 a sa concavité tournée vers les parties 21.

Le pontet sécable 25 de chaque branche 2 est délimité par deux entailles 27 en forme de pointes, symétriques l'une de l'autre par rapport au plan moyen π de l'agrafe et creusées dans la direction de profondeur Z de la branche 2. Comme montré sur la figure 3, chaque entaille 27 définit deux surfaces internes S₁ et S₂ de la branche 2, adjacentes respectivement à la partie 21 et à la partie 23 de la branche 2. Les deux surfaces S₂ d'une branche 2 sont convergentes en direction de la partie 21 de la branche et inclinées selon un angle de l'ordre de 45° par rapport à l'axe longitudinal X₂ de la branche, alors que les deux surfaces S₁ d'une branche 2 sont comprises sensiblement dans un même plan, qui, dans la configuration initiale de l'agrafe 1, est parallèle à la direction Z de l'agrafe 1 et incliné selon un angle β de l'ordre de 15° par rapport à la direction Y de l'agrafe. Ainsi, la surface de coupure S₂₅ du pontet sécable 25, qui correspond à la section d'épaisseur minimale e₂₅ du pontet 25, est également comprise dans un plan parallèle à la direction Z et inclinée selon un angle β de l'ordre de 15° par rapport à la direction Y lorsque l'agrafe 1 est dans sa configuration initiale.

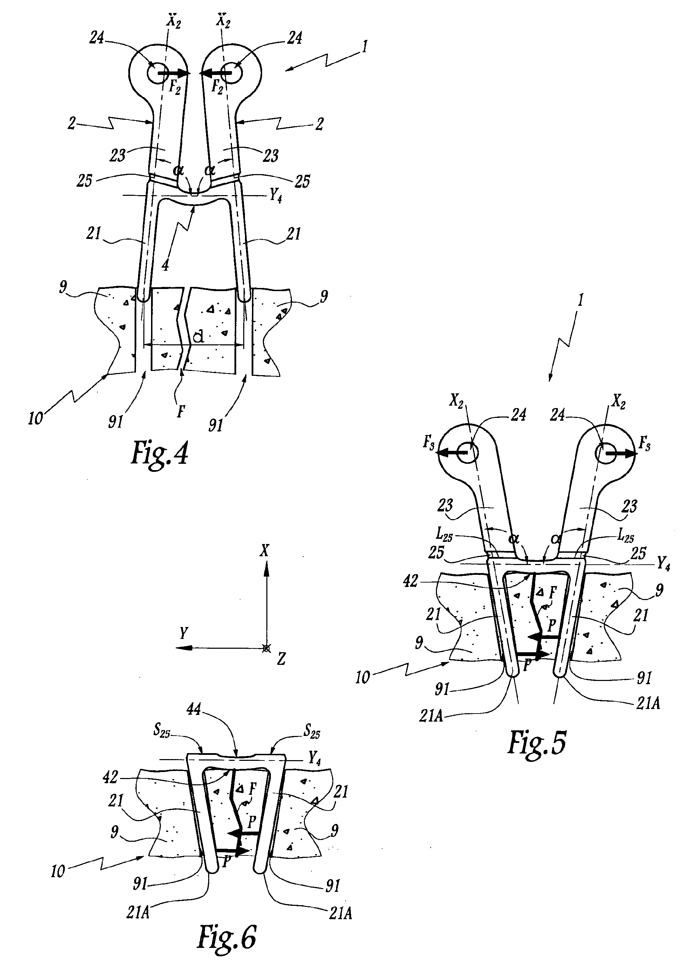

On note L₂₅ la trace, sur la figure 2, de la surface de coupure S₂₅ de chaque branche 2. Dans la configuration initiale de l'agrafe 1, chaque trace L₂₅ est inclinée selon l'angle β par rapport à la direction Y de l'agrafe, de telle sorte que les traces L₂₅ des surfaces de coupure S₂₅ des deux branches 2 sont convergentes en direction du pont 4 et agencées dans la continuité du bord 44 du pont 4 dirigé du côté des parties 23 des branches 2. Une telle configuration des surfaces de coupure S₂₅ dans la configuration initiale de l'agrafe 1 garantit que, en configuration inclinée des deux branches 2 par rapport à leur position initiale pour faire converger les parties 21, la surface de coupure S₂₅ de chaque branche 2 est comprise dans un plan sensiblement parallèle aux directions Y et Z de l'agrafe, ce plan étant agencé au même niveau que les extrémités du bord 44 du pont 4, comme montré sur la figure 5.

Comme visible sur la figure 2, l'extrémité 21B de la partie 21 de chaque branche 2 a une section transversale σ₁, prise perpendiculairement à l'axe longitudinal X₂ de la branche, supérieure à la section transversale σ₂, prise perpendiculairement à l'axe X₂, du reste de la partie 21. Ainsi, la portion 21B de liaison entre le pont 4 et la partie 21 de chaque branche 2, qui est soumise à des efforts importants lors de l'implantation de l'agrafe, est renforcée et apte à supporter des contraintes locales résultant du déplacement en pivotement des branches 2 par rapport au pont 4. La section transversale de la partie 23 de chaque branche 2 est choisie égale à la section transversale σ₁ de l'extrémité 21B, de sorte que chaque branche 2 n'est pas déformable à la liaison entre les parties 21 et 23 lors de l'actionnement du pivotement de la branche 2 par l'application d'un effort F₂, F₃ sur l'extrémité libre 23B de la partie 23. En vue de l'application d'un tel effort F₂, F₃ d'entraînement en pivotement d'une branche 2, la partie 23 de chaque branche 2 est pourvue, au voisinage de son extrémité libre 23B, d'un orifice 24 propre à recevoir un organe d'actionnement du pivotement de la branche.

Une méthode de pose de l'agrafe de compression 1 pour la consolidation de deux parties osseuses 9 d'un os 10 fracturé ou ayant subi une ostéotomie, par exemple une phalange ou un métatarse, comprend des étapes dans lesquelles :

Tout d'abord, on perce un alésage 91 dans chacune des deux parties osseuses 9, de part et d'autre du foyer F de fracture ou d'ostéotomie de l'os 10. De manière avantageuse, en configuration des parties osseuses 9 plaquées l'une contre l'autre sans compression, comme montré schématiquement sur la figure 4, les alésages 91 sont distants l'un de l'autre d'une distance d supérieure à l'entraxe a entre les parties 21 des branches 2 dans la configuration initiale de l'agrafe 1, dans laquelle les branches 2 sont parallèles l'une à l'autre.

On procède alors à l'introduction des parties 21 des branches 2 de l'agrafe à l'intérieur des alésages 91. A cet effet, on opère un pivotement de chacune des branches 2 par rapport au pont 4 parallèlement au plan moyen π, de manière à faire diverger les parties 21 des deux branches 2 l'une par rapport à l'autre en s'éloignant du pont 4, par l'application d'un effort F₂ de convergence sur les extrémités libres 23B des deux branches 2, au niveau des orifices 24. Les parties 21 sont ainsi amenées dans une configuration divergente dans laquelle les extrémités libres 21 A sont distantes de la distance d. Les parties 21 sont alors aptes à être insérées à l'intérieur des alésages 91 des parties osseuses 9.

La divergence des parties 21 l'une par rapport à l'autre permet de faciliter l'introduction des parties 21 à l'intérieur des alésages 91 des parties osseuses 9. Toutefois, cette divergence est annulée dès que les parties 21 pénètrent dans les alésages 91. Selon une variante non représentée de l'invention, les alésages 91 peuvent être percés en étant distants l'un de l'autre, en configuration des parties osseuses 9 plaquées l'une contre l'autre, d'une distance d égale à l'entraxe a entre les parties 21 des branches 2 dans la configuration initiale de l'agrafe 1. La divergence des parties 21 l'une par rapport à l'autre préalablement à leur insertion dans les alésages 91 n'est alors plus nécessaire.

Au cours de l'insertion des parties 21 à l'intérieur des alésages 91, l'agrafe 1 revient progressivement, par exemple sous l'action d'un outil agissant au niveau des orifices 24, vers sa configuration initiale, c'est-à-dire vers une configuration dans laquelle les branches 2 sont parallèles l'une à l'autre. Ce retour à la configuration initiale de l'agrafe 1 est imposé lors de l'enfoncement des parties 21 dans les alésages 91. L'enfoncement des parties 21 est réalisé jusqu'à la venue en contact d'un bord 42 du pont 4, tourné du côté des parties 21, contre la surface de l'os 10. Au fur et à mesure de l'insertion des parties 21 dans les alésages 91, les parties osseuses 9 sont appliquées plus fermement l'une contre l'autre, du fait du retour des branches 2 vers une configuration dans laquelle elles sont parallèles l'une à l'autre.

Lorsque les parties 21 ont été enfoncées dans les alésages 91 de manière suffisante pour obtenir la venue en contact du bord 42 du pont 4 contre la surface de l'os 10, on opère un nouveau pivotement des branches 2 par rapport au pont 4, parallèlement au plan moyen π, de telle sorte que les parties 21 convergent l'une vers l'autre en s'éloignant du pont 4. A cet effet, on applique un effort F₃ de divergence sur les extrémités libres 23B des parties 23, au niveau des orifices 24. Le rapprochement des extrémités libres 21 A des parties 21 génère un effort de rapprochement P des parties osseuses 9, ce qui permet d'appliquer l'une contre l'autre les surfaces des deux parties osseuses, au niveau du foyer F, avec une certaine pression. Grâce aux propriétés mécaniques du matériau constitutif de l'agrafe 1, les parties 21 des branches 2 restent convergentes malgré l'effort de réaction exercé par la paroi de chaque alésage 91 sur la partie 21 correspondante.

On procède alors à la séparation de la partie 23 de chaque branche 2 par rapport à la partie 21 adjacente, en exerçant un effort de poussée et de traction dirigé transversalement par rapport au plan moyen π de l'agrafe 1 sur l'extrémité libre 23B de la partie 23, comme montré par la flèche F₄ de la figure 1. L'effort F₄ de traction et de poussée est exercé sur la partie 23 de la branche 2 jusqu'à la rupture du pontet sécable 25 de la branche 2. Lorsque la partie 23 de chaque branche 2 a été coupée par rapport à la partie 21 correspondante, l'agrafe 1 est dans la configuration implantée montrée sur la figure 6, dans laquelle chaque surface de coupure S₂₅ est sensiblement parallèle et confondue avec le plan du bord 44 du pont 4, lequel a été déformé lors de l'application de l'effort de compression aux parties osseuses 9.

Comme visible sur la figure 6, l'agrafe 1 en configuration implantée dans l'os 10, c'est-à-dire après séparation des parties 23 par rapport aux parties 21 des branches, présente un profil bas et compact à la surface de l'os. De manière particulièrement avantageuse, la surface de l'agrafe 1 qui est dirigée à l'opposé de l'os 10, laquelle est formée par le bord 44 du pont 4 et les surfaces de coupure S₂₅ des parties 21, ne présente pas de portion saillante ou agressive, grâce à la conception optimisée de l'agrafe 1. En particulier, les pontets sécables 25 des branches 2 ont des caractéristiques adaptées, en terme d'inclinaison notamment, pour ne pas générer de saillies par rapport au bord supérieur 44 du pont 4 suite à la découpe des parties 23, en configuration convergente des parties 21. La présence des parties sécables 23 d'entraînement des branches 2 en déplacement par rapport au pont 4 permet de ne pas utiliser le pont 4 pour l'application d'un effort de rapprochement sur les branches. Dès lors, ce pont 4 peut présenter un encombrement limité à la surface de l'os, ce qui réduit les risques de gêne sous la peau et de détérioration des tissus au voisinage de l'agrafe.

De plus, comme il ressort de l'exemple décrit ci-dessus, il est possible de maîtriser la charge de compression appliquée aux parties osseuses 9, par l'application d'un effort de divergence F₃ adapté sur les parties 23 des branches 2. Il est également possible de revenir en arrière par rapport au mouvement de convergence de parties 21 lors de l'application de la compression au parties osseuses 9, en particulier lorsque la compression génère un déplacement non désiré. Cette déformation contrôlée de l'agrafe 1 conforme à l'invention se distingue de la déformation obtenue avec les agrafes de l'état de la technique constituées en matériau à mémoire de forme, pour lesquelles la charge de compression appliquée aux parties osseuses à consolider est imposée par les caractéristiques du matériau à mémoire de forme, sans possibilité de contrôle lors de l'implantation de l'agrafe. En outre, une agrafe de compression conforme à l'invention, constituée par exemple en acier inoxydable ou en titane, est moins susceptible de donner lieu à des réactions allergiques qu'une agrafe constituée en matériau à mémoire de forme, et n'impose pas de contraintes de température pour son stockage et sa mise en oeuvre.

Par ailleurs, grâce à la présence des parties 23 des branches 2, qui permettent l'entraînement en pivotement des branches 2 dans le plan moyen π de l'agrafe par rapport au pont 4, la compression peut être appliquée sur les parties osseuses lorsque l'agrafe 1 est déjà enfoncée au maximum dans l'os, c'est-à-dire lorsque le pont 4 est en contact avec la surface de l'os. Dès lors, il n'est pas nécessaire de procéder à une impaction ultérieure de l'agrafe après rapprochement des branches, ce qui est avantageux.

Enfin, une agrafe de compression conforme à l'invention, constituée par exemple en acier inoxydable, peut être fabriquée aisément par découpe laser, découpe au jet d'eau, ou autre, avec un coût de fabrication limité.

L'invention n'est pas limitée à l'exemple décrit et représenté. En particulier, une agrafe de compression conforme à l'invention peut présenter une forme différente de celle représentée sur les figures. Le pont 4 peut notamment présenter une forme rectiligne, au lieu d'être bombé du côté des parties 21 des branches. La partie proximale de la partie 21 de chaque branche 2, destinée à être ancrée dans un os, peut également présenter une section plus grande pour éviter la flexion de la branche.

Par ailleurs, les moyens sécables de raccordement entre les première et deuxième parties d'une agrafe de compression selon l'invention peuvent être de toute type approprié autre qu'un pontet sécable. Lorsque les moyens sécables sont formés par un pontet sécable, ce pontet peut également présenter des caractéristiques différentes de celles du pontet 25 décrit précédemment, par exemple en termes de localisation ou d'inclinaison par rapport aux branches.

Une agrafe de compression conforme à l'invention peut également être constituée en tout matériau adapté autre qu'un acier inoxydable. En particulier, une agrafe de compression selon l'invention, présentant des parties sécables 23 d'entraînement des branches en pivotement par rapport au pont, peut être constituée en un matériau à mémoire de forme. La présence des parties 23 permet alors de compenser un éventuel retour du matériau à mémoire de forme vers une configuration convergente des parties 21 en cas de variations de température avant l'implantation de l'agrafe.

Par ailleurs, il est possible d'omettre certaines étapes de la méthode décrite précédemment pour la pose d'une agrafe selon l'invention. En particulier, une agrafe de compression selon l'invention peut être implantée dans un os dans sa configuration initiale, sans mouvement de divergence préalable des parties 21 de ses branches. La distance d entre les alésages des parties osseuses est alors choisie sensiblement égale à l'entraxe a entre les parties 21 dans la configuration initiale de l'agrafe. De plus, en fonction du profil des parties osseuses à consolider, il peut ne pas être possible de faire converger les parties 21 des branches lors de l'application de l'effort de compression aux parties osseuses. Dans ce cas, un effort de divergence F₃ est néanmoins appliqué aux parties 23 des branches, de manière à solliciter les parties 21 en convergence l'une vers l'autre.

Selon une variante non représentée de l'invention, une agrafe de compression conforme à l'invention peut en outre être munie de moyens de maintien des parties 21 de l'agrafe en configuration ancrée dans les parties osseuses, de manière à éviter toute sortie de l'agrafe par rapport à l'os. Ces moyens de maintien peuvent être formés par des dents ou des rainures ménagées sur une face externe ou interne des parties 21, ou encore par des organes de crochetage, agencés de préférence au voisinage des extrémités distales 21 A ou proximales 21B des parties 21.

## Revendications

1. Agrafe de compression (1), du type comprenant deux branches (2) reliées par un pont transversal (4), **caractérisée en ce que** chaque branche (2) comporte une première (21) et une deuxième (23) parties agencées dans le prolongement l'une de l'autre et raccordées l'une à l'autre par des moyens sécables (25), la première partie (21) étant une partie d'ancrage de la branche (2) dans un os (9), alors que la deuxième partie (23) est une partie d'entraînement de la branche (2) en déplacement par rapport au pont (4).

2. Agrafe de compression selon la revendication 1, **caractérisée en ce que** la deuxième partie (23) est une partie d'entraînement de la branche (2) en déplacement par rapport au pont (4) parallèlement à un plan moyen (π) défini par l'axe longitudinal (X₂) de la branche (2) et l'axe longitudinal (Y₄) du pont (4).

3. Agrafe de compression selon l'une quelconque des revendications 1 ou 2, **caractérisée en ce que** le pont (4) est lié à chaque branche (2) au niveau d'une extrémité (21 B) de la première partie (21) adjacente à la deuxième partie (23).

4. Agrafe de compression selon l'une quelconque des revendications précédentes, **caractérisée en ce que** les première (21) et deuxième (23) parties de chaque branche (2) sont raccordées par des moyens (25) sécables sous l'action d'un effort (F₄) dirigé transversalement par rapport à un plan moyen (π) défini par l'axe longitudinal (X₂) de la branche (2) et l'axe longitudinal (Y₄) du pont (4).

5. Agrafe de compression selon la revendication 4, **caractérisée en ce que** les moyens sécables de chaque branche (2) comprennent un pontet sécable (25) entre les première (21) et deuxième (23) parties ayant une épaisseur (e₂₅), selon une direction (Z) transversale par rapport au plan moyen (π), inférieure à l'épaisseur (e₂₁, e₂₃) des première et deuxième parties selon cette direction (Z).

6. Agrafe de compression selon la revendication 5, **caractérisée en ce que** le pontet sécable (25) de chaque branche (2) est défini par au moins une entaille (27) de la branche (2).

7. Agrafe de compression selon l'une quelconque des revendications précédentes, **caractérisée en ce que** ies première (21) et deuxième (23) parties de chaque branche (2) sont raccordées par des moyens (25) sécables sous l'action d'un effort (F₄) exercé sur une partie de la branche parmi les premières et deuxième parties, alors que l'autre partie de la branche est immobilisée.

8. Agrafe de compression selon l'une quelconque des revendications précédentes, **caractérisée en ce que** les moyens sécables (25) de chaque branche (2) définissent une surface de coupure (S₂₅) entre les première (21) et deuxième (23) parties de la branche.

9. Agrafe de compression selon la revendication 8, **caractérisée en ce que** la surface de coupure (S₂₅) de chaque branche (2) est agencée, en configuration convergente de la première partie (21) de la branche (2) vers la première partie (21) de l'autre branche (2), au même niveau qu'au moins une partie d'un bord (44) du pont (4) dirigé du côté de la deuxième partie (23) de chaque branche (2).

10. Agrafe de compression selon l'une quelconque des revendications précédentes, **caractérisée en ce que** l'extrémité (21 B) de la première partie (21) de chaque branche (2) adjacente à la deuxième partie (23) de la branche a une section transversale (σ₁), perpendiculairement à l'axe longitudinal (X₂) de la branche, supérieure à la section transversale (σ₂) du reste de la première partie (21), perpendiculairement à l'axe longitudinal (X₂) de la branche.

11. Agrafe de compression selon l'une quelconque des revendications précédentes, **caractérisée en ce que**, préalablement à l'implantation de l'agrafe (1) dans un os, le pont (4) est bombé du côté des premières parties (21) des deux branches (2).

12. Agrafe de compression selon l'une quelconque des revendications précédentes, **caractérisée en ce qu'**elle est formée par une plaque métallique découpée globalement selon une forme de H, chaque branche (2) du H étant apte à pivoter par rapport au pont transversal (4) du H parallèlement au plan moyen (π) de la plaque.

13. Agrafe de compression selon l'une quelconque des revendications précédentes, **caractérisée en ce que** la deuxième partie (23) de chaque branche (2) comporte un orifice (24) de réception d'un organe d'actionnement du déplacement de la branche (2) par rapport au pont (4) parallèlement à un plan moyen (π) défini par l'axe longitudinal (X₂) de la branche (2) et l'axe longitudinal (Y₄) du pont (4).

14. Agrafe de compression selon l'une quelconque des revendications précédentes, **caractérisée en ce que** la première partie (21) de chaque branche (2) comporte des moyens de maintien de la première partie (21) en configuration ancrée dans un os (9).

## Claims

1. Compression staple (1) of the type comprising two branches (2) connected by a transverse bridge (4), **characterised in that** each branch (2) comprises a first part (21) and a second part (23) arranged so as to extend from one another and connected by separable means (25), the first part (21) being used to anchor the branch (2) in a bone (9), whereas the second part (23) is used to guide the branch (2) in displacement relative to the bridge (4).

2. Compression staple according to claim 1, **characterised in that** the second part (23) is used to guide the branch (2) in displacement relative to the bridge (4) and parallel to a median plane (π) defined by the longitudinal axis (X₂) of the branch (2) and the longitudinal axis (Y₄) of the bridge (4).

3. Compression staple according to either claim 1 or claim 2, **characterised in that** the bridge (4) is connected to each branch (2) at an end (21B) of the first part (21) adjacent to the second part (23).

4. Compression staple according to any one of the preceding claims, **characterised in that** the first part (21) and second part (23) of each branch (2) are connected by separable means (25) which can be broken off by exerting a force (F₄) transverse to the median plane (π) defined by the longitudinal axis (X₂) of the branch (2) and the longitudinal axis (Y₄) of the bridge (4).

5. Compression staple according to claim 4, **characterised in that** the separable means of each branch (2) comprise a separable bridging connection (25) between the first part (21) and the second part (23) having a thickness (e₂₅), in a transverse direction (Z) relative to the median plane (π), which is smaller than the thickness (e₂₁, e₂₃) of the first and second parts in said direction (Z).

6. Compression staple according to claim 5, **characterised in that** the separable bridging connection (25) of each branch (2) is defined by at least one notch (27) in the branch (2).

7. Compression staple according to any one of the preceding claims, **characterised in that** the first part (21) and second part (23) of each branch (2) are connected by separable means (25) which can be broken off by exerting a force (F₄) on either the first part or the second part of the branch, whilst the other part of the branch remains still.

8. Compression staple according to any one of the preceding claims, **characterised in that** the separable means (25) of each branch (2) define a cutting face (S₂₅) between the first part (21) and second part (23) of the branch.

9. Compression staple according to claim 8, **characterised in that** the cutting face (S₂₅) of each branch (2) is arranged so the first part (21) of the branch (2) is inclined towards the first part (21) of the other branch (2) at the same height as at least a part of an edge (44) of the bridge (4) on the side of the second part (23) of each branch (2).

10. Compression staple according to any one of the preceding claims, **characterised in that** the end (21B) of the first part (21) of each branch (2) adjacent to the second part (23) of the branch has a cross-section (σ₁), perpendicular to the longitudinal axis (X₂) of the branch, which is greater than the cross-section (σ₂) of the rest of the first part (21) perpendicular to the longitudinal axis (X₂) of the branch.

11. Compression staple according to any one of the preceding claims, **characterised in that** before the clip (1) is implanted in a bone the bridge (4) is curved outwardly on the side of the first parts (21) of the two branches (2).

12. Compression staple according to any one of the preceding claims, **characterised in that** it is formed by a metal plate cut basically into an H shape, each branch (2) of the H being able to pivot relative to the transverse bridge (4) of the H and parallel to the median plane (π) of the plate.

13. Compression staple according to any one of the preceding claims, **characterised in that** the second part (23) of each branch (2) comprises an aperture (24) for receiving an element for actuating displacement of the branch (2) relative to the bridge (4) and parallel to a median plane (π) defined by the longitudinal axis (X₂) of the branch (2) and the longitudinal axis (Y₄) of the bridge (4).

14. Compression staple according to any one of the preceding claims, **characterised in that** the first part (21) of each branch (2) comprises means for holding the first part (21) in place when anchored in a bone (9).

## Patentansprüche

1. Kompressionsklammer von der Art, die zwei Schenkel aufweist, welche durch eine Querbrücke (4) verbunden sind, **dadurch gekennzeichnet, dass** jeder Schenkel (2) einen ersten (21) und einen zweiten (23) Teil aufweist, wobei der eine Teil in Verlängerung des anderen Teils angeordnet ist und die Teile miteinander durch trennbare Mittel (25) verbunden sind und wobei der erste (21) ein Teil zur Verankerung des Schenkels (2) in einem Knochen (9) ist, während der zweite Teil (23) ein Antriebsteil zum Verschieben des Schenkel (2) relativ zur Brücke ist

2. Kompressionsklammer nach Anspruch 1, **dadurch gekennzeichnet, dass** der zweite Teil (23) als Antriebsteil des Schenkels (2) durch Verschieben relativ zur Brücke (4) und parallel zu einer Mittelebene (π), die von der Längsachse (X₂) des Schenkels (2) und der Längsachse (Y₄) der Brücke (4) definiert wird, dient.

3. Kompressionsklammer nach einem der Ansprüche 1 oder 2, **dadurch gekennzeichnet, dass** die Brücke (4) mit jedem Schenkel (2) in Höhe eines Endes (21B) des ersten Teils (21) verbunden ist, das dem zweiten Teil (23) benachbart ist.

4. Kompressionsklammer nach einem der vorhergehenden Ansprüche, **dadurch gekennzeichnet, dass** der erste (21) und der zweite (23) Teil jedes Schenkels (2) über Mittel (25) verbunden sind, die unter der Wirkung einer Kraft (F₄), die bezüglich einer von der Längsachse (X₂) des Schenkels (2) und der Längsachse (Y₄) der Brücke (4) definierten Mittelebene (π) quer gerichtet ist, trennbar sind.

5. Kompressionsklammer nach Anspruch 4, **dadurch gekennzeichnet, dass** die trennbaren Mittel jedes Schenkels (2) einen trennbaren Steg (25) zwischen dem ersten (21) und dem zweiten (23) Teil aufweisen mit einer Dicke (e₂₅) in einer Richtung (Z) quer zur Mittelebene (π) geringer als die Dicke (e₂₁, e₂₃) des ersten und zweiten Teils gemäß dieser Richtung (Z).

6. Kompressionsklammer nach Anspruch 5, **dadurch gekennzeichnet, dass** der trennbare Steg (25) jedes Schenkels (2) durch mindestens einen Einschnitt (27) des Schenkels (2) definiert wird.

7. Kompressionsklammer nach einem der vorhergehenden Ansprüche, **dadurch gekennzeichnet, dass** der erste (21) und der zweite (23) Teil jedes Schenkels (2) durch Mittel (25) verbunden sind, die unter der Wirkung einer Kraft (F₄) trennbar sind, welche auf einen Abschnitt des ersten und zweiten Teils des Schenkels ausgeübt wird, während der andere Teil des Schenkels blockiert ist.

8. Kompressionsklammer nach einem der vorhergehenden Ansprüche, **dadurch gekennzeichnet, dass** die trennbaren Mittel (25) jedes Schenkels (2) eine Schnittfläche (S₂₅) zwischen dem ersten (21) und dem zweiten (23) Teil des Schenkels definieren.

9. Kompressionsklammer nach Anspruch 8, **dadurch gekennzeichnet, dass** die Schnittfläche (S₂₅) jedes Schenkels (2) in einer vom ersten Teil (21) des Schenkels (2) zum ersten Teil (21) des anderen Schenkels (2) konvergierenden Konfiguration auf der gleichen Höhe wie mindestens ein Teil eines Rands (44) der Brücke (4) angeordnet ist, der zur Seite des zweiten Teils (23) jedes Schenkels (2) weist.

10. Kompressionsklammer nach einem der vorhergehenden Ansprüche, **dadurch gekennzeichnet, dass** das Ende (21B) des ersten Teils (21) jedes Schenkels (2), das an den zweiten Teil (23) des Schenkels angrenzt, einen Querschnitt (σ₁) lotrecht zur Längsachse (X₂) des Schenkels hat, der größer ist als der Querschnitt (σ₂) des Rests des ersten Teils (21) lotrecht zur Längsachse (X₂) des Schenkels.

11. Kompressionsklammer nach einem der vorhergehenden Ansprüche, **dadurch gekennzeichnet, dass** vor dem Einsetzen der Klammer (1) in einen Knochen die Brücke (4) zur Seite der ersten Teile (21) der zwei Schenkel (2) gewölbt ist.

12. Kompressionsklammer nach einem der vorhergehenden Ansprüche, **dadurch gekennzeichnet, dass** sie von einer Metallplatte geformt wird, die im Wesentlichen in Form eines H ausgeschnitten wird, wobei jeder Schenkel (2) des H bezüglich der Querbrücke (4) des H parallel zur Mittelebene (π) der Platte schwenkbar ausgebildet ist.

13. Kompressionsklammer nach einem der vorhergehenden Ansprüche, **dadurch gekennzeichnet, dass** der zweite Teil (23) jedes Schenkels (2) eine Öffnung (24) für die Aufnahme eines Betätigungsorgans zur Verschiebung des Schenkels (2) bezüglich der Brücke (4) parallel zu einer Mittelebene (π) aufweist, die von der Längsachse (X₂) des Schenkels (2) und der Längsachse (Y₄) der Brücke (4) definiert wird.

14. Kompressionsklammer nach einem der vorhergehenden Ansprüche, **dadurch gekennzeichnet, dass** der erste Teil (21) jedes Schenkels (2) Einrichtungen zum Halten des ersten Teils (21) in der in einem Knochen (9) verankerten Stellung aufweist.
